Europäisches Patentamt

European Patent Office

Office européen des brevets

(11) Publication number: **0 249 308**
**A2**

# EUROPEAN PATENT APPLICATION

(21) Application number: 87302400.4

(51) Int. Cl.4: **A61M 1/14**

(22) Date of filing: **19.03.87**

(30) Priority: **13.06.86 US 874288**

(43) Date of publication of application:
**16.12.87 Bulletin 87/51**

(84) Designated Contracting States:
**DE FR GB IT**

(71) Applicant: **BAXTER TRAVENOL LABORATORIES, INC.**
**One Baxter Parkway**
**Deerfield, IL 60015(US)**

(72) Inventor: **Sutherland, Karl M.**
**16141 Osborn Street**
**Westminster California 92683(US)**
Inventor: **Jenusaitis, Matthew**
**22 Morningstar Avenue**
**Irvine California 92715(US)**
Inventor: **Pearson, Derek**
**Wayside 53 The Grove**
**Gosforth Newcastle Upon Tyne, NE3 1NJ(GB)**

(74) Representative: **Orchard, Oliver John**
**JOHN ORCHARD & CO. Staple Inn Buildings**
**North High Holborn**
**London WC1V 7PZ(GB)**

(54) O2/CO2 control in blood oxygenators.

(57) A blood oxygenator for mixing blood and oxygen to absorb the oxygen into the blood and release carbon dioxide from the blood is described. The oxygenator includes first and second oxygen inlets associated with the oxygenator and a variable gas flow device associated with both the first and second oxygen inlets for allowing the amount of oxygen flowing from each of the inlets to the oxygenator to vary and thereby independently control oxygen and carbon dioxide transfer.

FIG. I.

## O₂/CO₂ CONTROL IN BLOOD OXYGENATORS

### BACKGROUND OF THE INVENTION

#### I. Field of the Invention

This invention relates to a system for gas concentration control, and more particularly to an apparatus such as a blood oxygenator which allows for the independent control of oxygen ($O_2$) and carbon dioxide ($CO_2$) transfer with regard to the blood without increasing the amount of $O_2$ that flows into the oxygenator via the oxygen inlet port so that during surgery the concentration of $O_2$ and $CO_2$ dissolved in the patients blood can be more precisely controlled.

#### II. Description of the Prior Art

In various types of surgical procedures it is often necessary to perform a treatment whereby the patients blood is subject to a bypass flow outisde of the patients body, and an apparatus such as an oxygenator is employed. Such oxygenators are used in open-heart surgery and other operations and treatments of the body when it is necessary to establish an extracorporeal circulation system for temporarily assuming the functions of the heart and lungs of the patient. In such a system the oxygenator operates to perform the function usually performed by the lungs of the patient, i.e., the life-supporting transfer of oxygen into the blood and carbon dioxide out of the blood. The oxygenator is used in association with a pump which performs the function of the heart to cause circulation of the blood. Thus, early versions of the oxygenator were often referred to as "heart-lung" machines. The early heart-lung machines were typically rotating discs which passed through a pool of blood, but were only partially immersed therein such that the free surface of the disc exposed the blood to oxygen and accomplished some gas transfer. After this, bag-type oxygenators were introduced which were superior to the disc oxygenators, but which left much to be desired.

At the present time two principle types of blood oxygenators are used which have proven efficient, provide minimal blood trauma, are convenient to set up and operate, are cost effective and have provided excellent clinical results, i.e. bubble oxygenators and membrane oxygenators. In a membrane oxygenator, a thin, highly gas permeable membrane is placed between the gas and blood. Venous blood flows along one side of the membrane and gas is on the other side. A concentration gradient is established so that when the partial pressure for oxygen is higher in the ventilating gas than the partial pressure for oxygen in the venous blood, oxygen will diffuse across the membrane into the blood. Bubble oxygenators simply diffuse gas bubbles into venous blood. The oxygenated blood is typically defoamed before it is ready for delivery to the patient.

The typical bubble oxygenator is constructed of three chambers that are connected in series with each other, i.e. (1) a gas exchange or bubble chamber in which gas is dispersed as bubbles into the venous blood through small holes in a distributing manifold or sparger that is particularly used to create bubbles of the proper diameter and to disperse them effectively in the venous blood i.e., create foam and bubbles, and an effective mixture of gas and blood such that transfer of the oxygen into the blod takes place; (2) a defoaming or debubbling chamber wherein after gas transfer is completed, coalescence of the foam and the removal of the remaining bubbles is performed; and (3) a settling chamber in which the defoamed and oxygenated blood settles prior to being pumped back to the patient. Typically a heat exchange element is used in the bubble chamber for maintaining the blood temperature as for hypothermia.

In all bubble type blood oxygenators two types of gas transfer must take place. One is oxygen ($O_2$) which is tranferrred into the blood and the other is carbon dioxide ($CO_2$ which is transferred out of the blood. Typically a bubble oxygenator provides an inlet means for oxygen and an outlet means for oxygen and carbon dioxide. In known bubble oxygenators there is one gas inlet, and if one wants to increase the partial pressure of the oxygen ($PO_2$), the gas flow to the oxygenator is increased. This is also true if one wants to decrease the $CO_2$, i.e. the gas flow to the inlet (flow of $O_2$ into the oxygenator) is increased. However, during, for example, open heart surgery when the patient is being cooled or is cooled down, it doesn't take as much oxygen to oxygenate the blood of the patient. The problem is that it is still desired to be able to remove or blow off $CO_2$, and in order to blow off more $CO_2$, it is necessary to provide a higher gas flow of $O_2$. Higher partial pressures of the $O_2$ are not wanted because of various undesirable side effects. It would therefore be desirable in a bubble oxygenator to be able to maintain a $CO_2$ transfer out of the blood while at the same time lowering the $O_2$ transfer into the blood.

Various prior art examples of blood oxygenators and gas-liquid type of transfer apparatus known in the art are described in U.S. Patent Numbers 3,065,748 (illustrates two outlets 16 and 17), 3,256,883, 3,493,347, (two inlets 18 and 29), 4,073,622, 4,138,288, 4,182,739, 4,203,944, 4,203,945, 4,288,125, 4,231,988, 4,272,373 (separate inlets for gas and water), 4,336,224, 4,370,151, 4,374,088, 4,396,584, 4,407,777, 4,440,722, 4,493,692 (two separate sources for $O_2$ and $O_2/CO_2$) and 4,533,516.

Examples of the bubble type blood oxygenator that can employ the features of the present invention are described in U.S. Patent Numbers 3,468,631, 3,488,158 and 3,578,411, the last two of which describe devices which have come to be known as the Bentley Oxygenator, and U.S. Patent Numbers 4,282,180 and 4,440,723, both assigned to Bentley Laboratories, Inc.

## SUMMARY OF THE INVENTION

It is therefore an object of the present invention to provide a system for controlling oxygen and carbon dioxide flow in an oxygenator which is substantially devoid of the above-noted disadvantages.

Another object of the present invention is to provide a system for controlling oxygen and carbon dioxide flow in an oxygenator which enables one to separately control oxygen and carbon dioxide flow independent of one another.

Still another object of the present invention is to provide a blood oxygenator which allows one to increase oxygen flow to the oxygenator without increasing the amount of oxygen transferred to the blood.

Still another object of the present invention is to provide a procedure to separately control oxygen and carbon dioxide independently in a bubble oxygenator by splitting the oxygen flow to the oxygenator.

The foregoing and other objects are accomplished in accordance with the features of the present invention by providing a blood oxygenator for mixing blood and oxygen to both absorb the oxygen into the blood and then release carbon dioxide from the blood. The blood oxygenator comprises a housing with a blood inlet means and a first and second oxygen inlet means. All of these inlet means are for introducing blood and oxygen into the housing. Variable gas flow means is provided to allow the amount of oxygen flowing from each of the inlets to vary and thereby independently control oxygen and carbon dioxide transfer. Also provided is a blood outlet means to provide an outlet for the oxygenated blood.

The first oxygen inlet feeds oxygen to a diffusing plate which forms bubbles of the oxygen and thereby helps to diffuse the oxygen into the blood. The second oxygen inlet feeds oxygen down through the housing to assist in carbon dioxide removal while avoiding transfer of the oxygen into the blood.

## BRIEF DESCRIPTION OF THE DRAWINGS

For a better understanding of the invention as well as other objects and further features thereof, reference is made to the following detailed disclosure of this invention taken in conjunction with the accompanying drawings wherein:

FIG. 1 is a plan sectional view of a bubble oxygenator taken along its axis which incorporates the features of the present invention and illustrates how the present invention is used with the oxygenator;

FIG. 2 is an enlarged plan sectional view of a bubble oxygenator as shown in FIG. 1 illustrating the flow of oxygen through the device; and

FIG. 3 is a plan sectional view of the type of gas splitter valve that is used with the bubble oxygenator shown in FIG. 1 to divert oxygen gas flow to the center of the oxygenator shown in FIG. 1.

## DESCRIPTION OF THE PREFERRED EMBODIMENTS

Since the system described by the present invention has particular use in a bubble type blood oxygenator, the various features of the present invention including its use will be described within the environment of a bubble type blood oxygenator.

A bubble oxygenator is generally cylindrical in configuration and comprises an outer shell which is provided with a gas inlet and a blood inlet. Other inlets and outlets are generally provided such as an inlet for priming as well as for optimal medication administration and, if needed, a return inlet from a cardiotomy reservoir. Blood outlets and a gas vent are also provided. There is also an inlet for heat exchanger fluid and an outlet for such fluid.

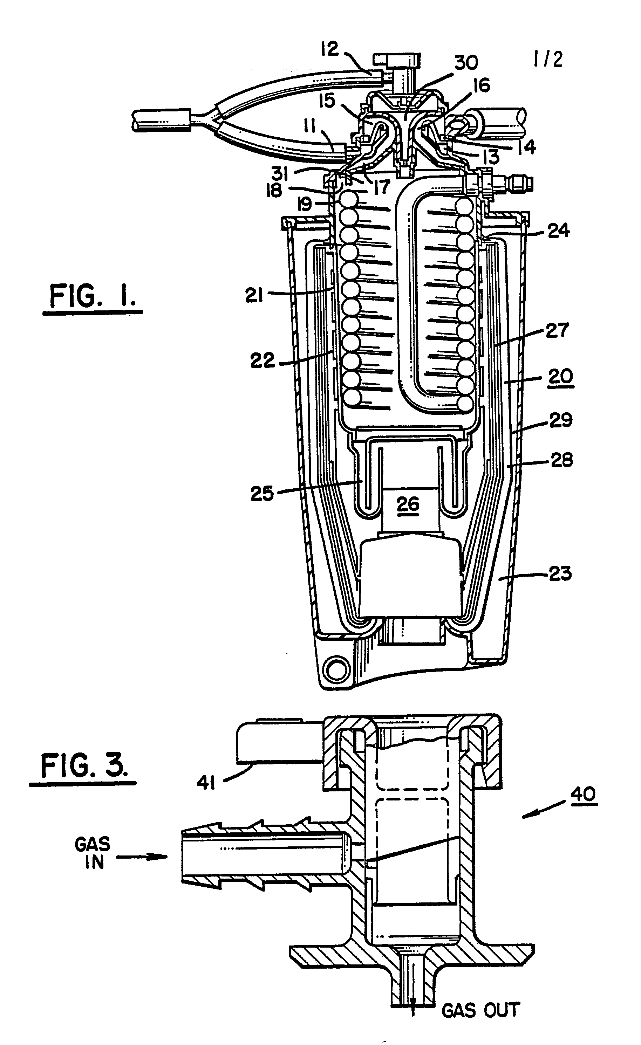

Referring now to FIG. 1, the internal construction of a bubble oxygenator 10 is shown in more detail. As illustrated, gas (oxygen) flows into the oxygenator through a gas inlet which in accordance with the features of the present invention consists of two gas inlets 11 and 12, the specific features and operation of which will be explained in detail hereinbelow. The gas inlet typically connects with annular chamber 13 which is bounded on its upper end by a sparger or diffusion means 14. This diffusion means 14 may be constructed of a plate of suitable porous material or a perforated apertured member, but preferably is a perforated member. An example of one type of diffusion means in the form of a perforated apertured member is a plate with three circular patterns containing 66 perforations which are 0.010 inches in diameter and 6 perforations which are 0.025 inches in diameter, the larger perforations being more or less uniformly distributed among the smaller perforations.

Blood inlet means (generally two inlet orifices connected to the very top portion of the oxygenator, which are not specifically shown in Figure 1) contact with the interior of annular chamber 15 in a generally tangential manner. Thus, when chamber 15 is filled with blood, flowing in a spiral manner, and gas is admitted to the device 10 through inlet 11, the gas, such as oxygen or an oxygen rich mixture, passes through inlet 11, into chamber 13 and through diffusion means 14 into the body of blood in chamber 15. Bubbles are formed in chamber 15 when the gas enters the blood.

Chamber 15 connects with annular channel 16 and undulating distribution channel 17, the latter generally being conical in shape. Channel 17 connects with annular mixing chamber 18 which is provided with heat exchange tubing 19. Heat exchange tubing 19 is preferrably a smooth and coiled helically wrapped tubing which provides for a helically descending flow path of the blood through the bubble column.

At the lower end of chamber 18, the outer wall of this chamber terminates approximately two-thirds of the distance from the top to the bottom of the oxygenator to permit the bubbles of blood to come into contact with defoaming means 20. While several defoaming means may be used, e.g., that disclosed in U.S. Patent No. 3,468,631, it is preferred in one embodiment of the present invention to form the defoaming material from a polyurethane foam having about ten to thirty pores per inch. The polyurethane foam and the other defoamer parts are coated with a silicone defoaming agent. Optionally, a spacer may be provided between defoaming material 20 and wall 21. The spacer may comprise a ribbed structure which provides open spaces therebetween.

Open spaces are provided in space 22 which permit blood to come into contact with defoaming material 20. The lower end of the oxygenator 10 is provided with reservoir 23 where oxygenated liquid blood is collected for subsequent return to the patient.

An annular passage (not shown) located on the very top portion of oxygenator 10 connects with a vent means (not shown) so that vent gases (e.g. oxygen or carbon dioxide) may be exhausted from the oxygenator. A mesh sleeve which may be of a polyester, polypropylene, polyethylene, nylon or other suitable fabric is positioned around defoaming material 20 and is provided with nylon bands 24 to hold it in place.

In order to obtain the desired gas transfer and be able to separately control oxygen transfer to the blood and carbon dioxide transfer out of the blood independently of one another in accordance with the features of the present invention, two separate gas inlet connectors 11 and 12 are connected to oxygenator 10 for the purpose of supplying oxygen or an oxygen-rich gas to the oxygenator. The first oxygen inlet connector 11 is preferably positioned in the so-called normal location for feeding oxygen to the oxygenator and the second oxygen inlet connector 12 is preferably positioned at the very top portion of oxygenator 10. The second oxygen inlet 12 preferably includes a valve type inlet that is able to vary the amount of gas that flows through the second inlet from about 0 (in which case all the oxygen gas will flow through the first oxygen inlet) to about 90 percent of the total gas flow to the oxygenator. During normal operation of oxygenator 10 all of the oxygen fed to the oxygenator is fed through the first oxygen inlet 11. When the demand for oxygen transfer to the blood is lowered, and the need for carbon dioxide transfer out of the blood is constant or increases (e.g. when the patient during surgery is on a bypass apparatus and his blood and body temperature is cooled), the oxygen is allowed to flow into both the first and second oxygen inlets 11 and 12 at the appropriate proportions by adjusting the valve. It should be noted that in accordance with the present invention, the proportion or split or percentage of gas flowing into each inlet will vary depending on the particular requirements for gas exchange. For example, this proportion can vary from about 100 percent gas through inlet 11 and 0 percent gas through inlet 12, to about 10 percent or less gas through inlet 11 to about 90 percent or more gas through inlet 12. The effect of this ratio becomes even greater if the amount of gas flow must be increased to obtain the desired carbon dioxide levels in the blood.

The operation of oxygenator 10 when employing the preferred features of the present invention is as follows: The first oxygen inlet 11 directs the oxygen which is fed through it into the oxygenator and through the apertures in diffusion means 14. The diffusion means then creates very small bubbles to mix with the incoming blood in chamber 15 thereby causing the blood to foam and oxygen to be transferred into the blood. The blood then flows through a torturous path which directs it down onto the heat exchanger tubing or coil 19. At this time the blood may be heated or cooled as desired. The blood continues to flow downward and through a gas expander chamber 25 and through a micro emboli inhibitor plug 26. Thereafter the blood travels preferably through a three stage defoamer 20 constructed of, for example, a polyester mesh 27, polyester foam 28 and a polyester tricot sock 29. The defoamer 20 separates the blood and gas allowing the gas to be vented off and the blood to enter reservoir 23 where it is then pumped back to the patient.

When the second oxygen inlet 12 to oxygenator 10 is opened via the valve connected thereto a portion of the oxygen flows into the valve and into the top portion of the oxygenator. This gas then flows into chamber 30 and then into chamber 31. What this causes is a reduced amount of oxygen being forwarded through the first oxygen inlet 11 for subsequent bubbling and mixing with the blood which results in a lower percent of oxygen transferred to the blood. At the same time the carbon dioxide transfer stays relatively unchanged since it is not as strongly associated with bubble size. Thus one can separately and independently control oxygen transfer and carbon dioxide transfer.

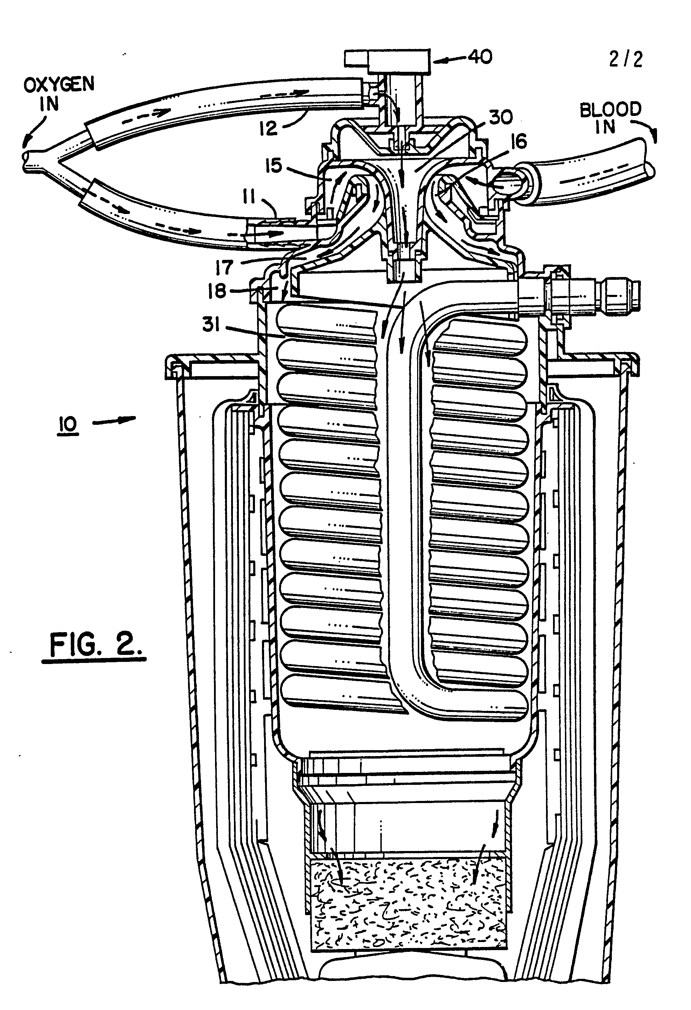

Specific details clearly illustrating the oxygen flow into and through the oxygenator 10 when oxygen is fed through a first oxygen inlet 11 and a second oxygen inlet 12 is clearly shown by the arrows in Figure 2 which represent oxygen flow.

The type of splitter mechanism which can be used with the features of the present invention to control the oxygen flow to inlets 11 and 12 and divert oxygen gas flow to the center of the oxygenator 10 is shown as splitter 40 in Figure 3. This function is achieved by using a small orifice that gradually opens as the handle 41 on top is turned. The amount of oxygen that can enter the oxygenator 10 is a function of the oxygen pressure, orifice size and back pressure in the unit.

While this invention has been described in conjunction with specific embodiments thereof, it is evident that many alternatives, modifications and variations will be apparent to those skilled in the art. Accordingly, the present invention is intended to embrace all such alternatives, modifications and variations, as fall within the spirit and scope of the appended claims.

## Claims

1. A blood oxygenator for mixing blood and oxygen to transfer oxygen into the blood and transfer carbon dioxide from the blood comprising: a housing; blood inlet means and first and second oxygen inlet means for introducing blood and oxygen into the housing; variable gas flow means to allow the amount of oxygen flowing from each of the first and second oxygen inlet means to the housing to vary and thereby independently control the oxygen and carbon dioxide transfer; and blood outlet means to provide an outlet for the oxygenated blood.

2. A blood oxygenator according to Claim 1 wherein said first oxygen inlet means feeds oxygen for diffusing into said blood and said second oxygen inlet means feeds oxygen down through said housing to assist in carbon dioxide removal from the blood while avoiding excessive transfer into said blood.

3. A blood oxygenator according to Claim 2 wherein said first oxygen inlet means feeds oxygen through a diffusion plate to create bubbles of oxygen which are mixed with said blood to transfer said oxygen into said blood.

4. A blood oxygenator according to Claim 1 wherein said second oxygen inlet means includes a valve for adjustment of the oxygen flow to said first and second inlets.

5. A blood oxygenator according to Claim 1 wherein said oxygenator is a bubble oxygenator.

6. A blood oxygenator according to Claim 2 wherein the amount of oxygen fed through said first oxygen inlet means varies from about 0 to about 100 percent of the total oxygen flow to said housing.

7. A blood oxygenator according to Claim 6 wherein the amount of oxygen fed through said second oxygen inlet means varies from about 0 to about 100 percent of the total oxygen flow to said housing.

8. A blood oxygenator according to Claim 1 wherein said variable gas flow means is a split valve.

9. A blood oxygenator according to Claim 1 wherein said first and second oxygen inlet means and said blood inlet means are connected to the top portion of said housing and feed said blood and oxygen into a downwardly extending blood-oxygen mixing chamber, only said first oxygen inlet means feeding oxygen for diffusion into said blood.

10. A blood oxygenator according to Claim 9 wherein heat exchange means are located in said mixing chamber.

11. A blood oxygenator according to Claim 10 wherein said heat exchange means constitutes a helically wrapped tube.

12. A blood oxygenator according to Claim 2 further comprising venting means to allow for the removal of gas from said oxygenator.

13. A blood oxygenator according to Claim 3 further comprising defoaming means for removing bubbles from said oxygen and blood mixture.

14. In a bubble type blood oxygenator wherein blood and oxygen are fed into an oxygenating chamber in a housing and mixed to absorb the oxygen into the blood and carbon dioxide is released from the blood, the improvement comprising means for separately controlling the oxygen and carbon dioxide transfer to and from the blood independently of one another.

15. A bubble type blood oxygenator according to Claim 14 wherein said separate control means includes a variable gas flow device for feeding oxygen to first and second oxygen inlet means thereby allowing transfer of oxygen to said blood and transfer of carbon dioxide from said blood to occur independently of one another.

16. A bubble type blood oxygenator according to Claim 15 wherein said first and second oxygen inlet means allows the oxygen to be fed to separate areas of said housing.

17. A bubble type blood oxygenator according to Claim 16 wherein only said first oxygen inlet means feeds oxygen for mixing with and diffusion into said blood.

18. A bubble type blood oxygenator according to Claim 17 wherein said second oxygen inlet means feeds oxygen to said housing to assist in carbon dioxide removal from said blood while avoiding excessive transfer into said blood.

19. A bubble type blood oxygenator according to Claim 16 wherein the amount of oxygen fed through said first oxygen inlet means varies from about 0 to about 100 percent of the total oxygen flow to said oxygenator.

20. A bubble type blood oxygenator according to Claim 19 wherein the amount of oxygen fed through said second oxygen inlet means varies from about 0 to about 100 percent of the total oxygen flow to said oxygenator.

21. A method of oxygenating blood and removing carbon dioxide from the blood while the blood is in a housing comprising the steps of:

(a) injecting oxygen gas into the housing through a first inlet to diffuse oxygen into the blood; and

(b) concurrently with step (a) injecting a separate and controlled amount of oxygen gas into the housing through a second inlet to control the carbon dioxide transfer from the blood independently of the oxygen flow of step (a).

FIG. 1.

FIG. 3.

GAS IN

GAS OUT

FIG. 2.